# FASCICULE DE BREVET EUROPEEN

(11) **EP 2 430 172 B1**
(45) Date de publication et mention de la délivrance du brevet: **08.04.2015**
(21) Numéro de dépôt: 10723170.6
(22) Date de dépôt: 06.05.2010
(51) Int. Cl.: C12P 7/10, C12P 17/04

(54) **PRODUCTION IN SITU DE FURFURAL EN QUANTITE CONTROLEE DANS UNE UNITE DE PRODUCTION D'ALCOOL À PARTIR DE BIOMASSE LIGNOCELLULOSIQUE**
IN-SITU-PRODUKTION VON FURFURAL IN EINER KONTROLLIERTEN MENGE IN EINER ALKOHOLPRODUKTIONSEINHEIT AUS EINER LIGNOCELLULOSE-BIOMASSE
IN SITU PRODUCTION OF FURFURAL IN A CONTROLLED AMOUNT IN AN ALCOHOL PRODUCTION UNIT FROM A LIGNOCELLULOSIC BIOMASS

(30) Priorité: 15.05.2009 FR 0902348
(43) Date de publication de la demande: 21.03.2012
(73) Titulaire: IFP Energies nouvelles, 92852 Rueil-Malmaison Cedex (FR)
(72) Inventeur: ROPARS, Marcel, F-91120 Palaiseau (FR); AYMARD, Caroline, F-69003 Lyon (FR); GHISONI, Flora, F-06640 Saint Jeannet (FR); MENIR, Sandra, F-95500 Gonesse (FR)
(86) Numéro de dépôt international: PCT/FR2010/000351
(87) Numéro de publication internationale: WO 2010/130889

(56) Documents cités:
- WO-A2-2009/102256
- OH, KYEONG KEUN ET AL.: "Pretreatment of Lignocellulosic Biomass using Combination ofAmmonia Recycled Percolation and Dilute-Acid Process", JOURNAL OF INDUSTRIAL AND ENGINEERING CHEMISTRY, vol. 8, no. 1, janvier 2002 (2002-01), pages 64-70, XP002569390, the Korean Society of Industrial and Engineering Chemistry, KOREA
- LARSSON S ET AL: "The generation of fermentation inhibitors during dilute acid hydrolyses of softwood", ENZYME AND MICROBIAL TECHNOLOGY, STONEHAM, MA, US, vol. 24, 1 janvier 1999 (1999-01-01), pages 151-159, XP003024061, ISSN: 0141-0229
- OHGREN K ET AL: "Simultaneous saccharification and co-fermentation of glucose and xylose in steam-pretreated corn stover at high fiber content with Saccharomyces cerevisiae TMB3400", JOURNAL OF BIOTECHNOLOGY, ELSEVIER SCIENCE PUBLISHERS, AMSTERDAM, NL, vol. 126, no. 4, 1 décembre 2006 (2006-12-01), pages 488-498, XP024956581, ISSN: 0168-1656 [extrait le 2006-12-01]
- CARVALHEIRO, FLORBELA ET AL.: "Hemicellulose biorefineries: a review on biomass pretreatments", JOURNAL OF SCIENTIFIC AND INDUSTRIAL RESEARCH, vol. 67, no. 11, novembre 2008 (2008-11), pages 849-864, XP002568985, ISSN: 0022-4456

## Description

### Domaine de l'invention

La présente invention s'inscrit dans le cadre d'un procédé de production d'alcool dit "de seconde génération" à partir de biomasse lignocellulosique.

### Étude de l'art antérieur

La biomasse lignocellulosique représente une des ressources renouvelables les plus abondantes sur terre, et certainement une des moins coûteuses. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux polymères : la cellulose (35 à 50%), l'hémicellulose (20 à 30%) qui est un polysaccharide essentiellement constitué de pentoses et d'hexoses et la lignine (15 à 25%) qui est un polymère de structure complexe et de haut poids moléculaire, provenant de la copolymérisation d'alcools phénylpropénoïques. Ces différentes molécules sont responsables des propriétés intrinsèques de la paroi végétale et s'organisent en un enchevêtrement complexe.

La cellulose et éventuellement les hémicelluloses sont les cibles de l'hydrolyse enzymatique mais ne sont pas directement accessibles aux enzymes. C'est la raison pour laquelle ces substrats doivent subir un prétraitement précédant l'étape d'hydrolyse enzymatique. Le prétraitement vise à modifier les propriétés physiques et physicochimiques du matériau lignocellulosique, en vue d'améliorer l'accessibilité de la cellulose insérée au sein de la matrice de lignine et d'hémicellulose.

De nombreuses technologies pour réaliser ce prétraitement existent : cuissons acides, cuissons alcalines, explosion à la vapeur, procédés organosolv, etc. L'efficacité du prétraitement se mesure à la fois par le bilan matière à l'issue du prétraitement (taux de récupération des sucres sous forme de monomères ou d'oligomères solubles ou de polymères solides) et également par la susceptibilité à l'hydrolyse des résidus cellulosiques et hémicellulosiques.

Le prétraitement acide en conditions douces et par explosion à la vapeur est très souvent utilisé puisqu'il permet une bonne récupération (supérieure à 90%) des pentoses et une bonne accessibilité de la cellulose à l'hydrolyse enzymatique. Il permet en effet de libérer les sucres contenus dans les hémicelluloses sous forme de monomères, essentiellement des pentoses, comme le xylose et l'arabinose, et des hexoses, comme le galactose, le mannose et le glucose. Ce type de prétraitement génère également des produits de dégradation des sucres qui sont principalement des aldéhydes tels que le furfural ou le 5-HMF (hydroxyméthyl furfural). Ces deux produits sont respectivement issus de la dégradation des pentoses ou des hexoses en milieu acide et à haute température. De nombreux autres produits tels que des acides organiques, des aldéhydes ou alcools phénoliques sont eux aussi issus des dégradations acides des sucres et de la lignine partiellement solubilisée.

Ces produits et tout particulièrement le furfural, qui correspond au produit de dégradation dont la concentration est le plus souvent la plus élevée, sont toxiques pour tous les micro-organismes et par conséquent pour la levure qui va transformer par fermentation les sucres en alcool. L'ensemble de ces composés toxiques sont dits "inhibiteurs" et ont pour origine un prétraitement réalisé à chaud en condition acide. On citera par exemple Klinke HB, Schmidt AS, Thomsen AB (1998) Biomass for Energy and Industry, Proceedings of the 10th European Conference and Technology Exhibition, CARMEN, Rimpar, Germany, 484-487, ou Palmquist et al. (2000) Biores. Technol. 74 : 1,17-24. Après ce type de prétraitement, les concentrations peuvent être comprises entre 3 à 5 g/L de furfural et 1 à 5 g/L de 5-HMF et ne sont pas contrôlées. La présence de furfural et de 5-HMF associée aux aldéhydes et alcools phénoliques présents entraînent des problèmes importants de toxicité. De nombreuses équipes travaillent actuellement sur la recherche de méthodes de détoxification qui pourraient faciliter la fermentation des sucres, tout particulièrement par les levures ou les bactéries alcooligènes. On peut citer par exemple l'article de Cantarella et al. publié dans Process Biochemistry (2004) vol 39, issue 11 p. 1533-1542.

Les sucres obtenus par hydrolyse de la biomasse lignocellulosique sont des pentoses (xylose et arabinose principalement), et des hexoses. Le glucose est le principal sucre disponible. Ce dernier est facilement transformé en éthanol par la levure S. *cerevisiae* utilisée par l'ensemble des industries de la fermentation alcoolique. Par contre, les pentoses ne sont fermentés que par des levures spécifiques (*P.stipitis, P.tannophilus*) et les performances sont médiocres.

La fermentation alcoolique en conditions non stériles présente un risque important de contamination du fermenteur par des micro-organismes opportunistes. Ces micro-organismes utilisent les nutriments présents dans le milieu, et peuvent être responsables de la formation de co-produits indésirables comme l'acide lactique ou l'acide acétique. On retrouve ce genre de micro-organismes partout où les conditions permettent leur croissance c'est-à-dire en présence d'un minimum de nutriments. Mentionnons ici que leurs exigences nutritionnelles sont : une source de carbone (généralement les sucres), une source d'acides aminés (constituants des protéines), certaines vitamines et oligo-éléments.

La contamination bactérienne est un problème majeur dans la production d'alcool par fermentation. Les bactéries présentes naturellement au sein de l'outil de production utilisent les nutriments présents dans le milieu, et entrent en concurrence avec les levures utilisées dans le procédé. La croissance et la viabilité des cellules de levures sont donc fortement affectées par la présence de ces bactéries et le rendement final en alcool en est également réduit.

Les bactéries lactiques comptent parmi les contaminants potentiels. En général, elles fermentent les sucres présents dans les moûts de fermentation et leur croissance est favorisée par des conditions anaérobies. Elles se développent généralement à un pH de 5,5 mais peuvent survivre à un pH aussi bas que pH 3,0. Ces bactéries opportunistes peuvent se développer sur une large gamme de température et sont tolérantes à de fortes concentrations en alcool dans le milieu. La présence des bactéries dans les procédés de production d'alcool de seconde génération est à proscrire.

Ainsi, dans le procédé actuel et non stérile de production d'éthanol de seconde génération, deux étapes sont sensibles à une éventuelle contamination microbiologique : l'étape d'hydrolyse enzymatique tout particulièrement et l'étape de fermentation. Les solutions connues actuellement pour lutter contre la contamination lactique lors de l'étape de fermentation consistent, par exemple, à abaisser le pH à une valeur favorisant le développement des levures au détriment des bactéries lactiques. Les levures sont cependant moins actives à ces pH acides. Ce type de solution n'est toutefois pas envisageable pour l'étape d'hydrolyse enzymatique. Une autre possibilité de lutte consiste à introduire des inhibiteurs de contamination bactérienne, comme par exemple des fluorures, des antibiotiques ou des sulfites. Ces procédures sont appliquées dans les procédés de production d'éthanol de première génération. L'utilisation d'anti-bactériens classiques a un coût relativement élevé et nécessite d'effectuer des relances assez fréquentes du processus de fermentation.

Contrairement aux prétraitements acides décrits dans l'art antérieur, le prétraitement alcalin selon la présente invention ne génère aucun des produits de dégradation des sucres communément obtenus dans les prétraitements acides. Ce type de prétraitement n'induit donc pas la fabrication de furfural. De ce fait, un hydrolysat issu d'un végétal préparé en conditions alcalines sera sensible aux infections bactériennes aussi bien dans l'étape d'hydrolyse enzymatique que dans l'étape de fermentation. Ce problème se rencontre également dans un procédé combinant simultanément les deux étapes d'hydrolyse enzymatique et de fermentation alcoolique (procédé SSF pour "Simultaneous Saccharification and Fermentation").

Limiter les risques de contamination constitue un gain potentiel de temps et d'argent pour des procédés industriels d'une telle ampleur et aucune solution n'est à négliger pour remédier à ce problème. Toute amélioration du procédé conduisant à une limitation maximale de cette contamination est à prendre en considération. La présente invention s'inscrit dans ce cadre et propose un procédé de production d'alcool avec prétraitement alcalin et protection anti-bactérienne.

### Résumé de l'invention

La présente invention décrit un procédé de production d'alcool dit "de seconde génération" à partir de biomasse lignocellulosique mettant en oeuvre un prétraitement alcalin et dans lequel on produit in-situ, à dose contrôlée, par conversion acide des pentoses issus directement du procédé, un agent anti-bactérien (furfural) destiné à protéger les sucres présents dans l'étape d'hydrolyse enzymatique et/ou de fermentation.

### Description détaillée de l'invention

Le procédé de production d'alcool à partir d'un substrat cellulosique ou lignocellulosique est un procédé dans lequel on produit du furfural et qui comprend au moins les étapes suivantes :
- une première étape de prétraitement chimique alcalin (étape A) dudit substrat, comprenant une étape de chauffage A1 en présence d'un réactif chimique alcalin, à l'issue de laquelle on obtient au moins un flux comprenant au moins ledit réactif chimique et le substrat prétraité et éventuellement une étape A2 de lavage dudit substrat prétraité, à l'issue de laquelle on obtient le substrat prétraité lavé et au moins un flux contenant les eaux de lavage usées, puis
- une étape de mise au pH entre 4,5 et 5,5 du substrat prétraité éventuellement lavé, couplée à une étape d'hydrolyse enzymatique (étape B1) dudit substrat prétraité mettant en jeu des enzymes cellulolytiques et/ou hémicellulolytiques,
- une étape de fermentation alcoolique (étape B2) de l'hydrolysat obtenu par un microorganisme alcooligène, à l'issue de laquelle on obtient un moût de fermentation comprenant des matières en suspension et une phase liquide comprenant un alcool et des vinasses, puis
- une étape de séparation/purification (étape C) à l'issue de laquelle on obtient l'alcool purifié, les vinasses clarifiées, et un résidu insoluble, et
- une étape de conversion acide (étape D) d'au moins un des liquides obtenus lors d'une des étapes précédentes et contenant des pentoses solubilisés, une partie desdits pentoses étant convertis en furfural, et
- une étape de mélange du furfural obtenu lors de l'étape de conversion acide avec le substrat prétraité éventuellement lavé lors de l'étape de mise au pH, la quantité de furfural mélangée étant comprise entre 1 et 5g/l par rapport à la quantité totale de liquide mise en jeu dans l'étape d'hydrolyse enzymatique et/ou de fermentation alcoolique.

Au sens de la présente invention, on désigne sous le terme de "pentose" les monomères et oligomères solubles des sucres comprenant 5 atomes de carbone.

Le procédé selon la présente invention propose donc une valorisation des pentoses, par transformation de ces composés en furfural. Par conséquent, le procédé selon la présente invention propose la production in-situ en quantité contrôlée d'un agent anti-bactérien destiné à protéger les différents sucres présents dans l'étape d'hydrolyse enzymatique et de fermentation alcoolique.

Ce procédé présente également l'avantage d'améliorer le rendement de conversion des sucres C6 en alcool, en limitant la production de glycérol, principal sous-produit de la fermentation alcoolique.

D'autre part, le mélange d'une solution acide comprenant du furfural produite par conversion des pentoses avec le substrat prétraité permet de façon très avantageuse de participer à la mise au pH nécessaire du substrat prétraité avant de réaliser l'hydrolyse enzymatique.

Le prétraitement chimique alcalin réalisé à l'étape A est préférentiellement un prétraitement de type Kraft à l'issue duquel on obtient des pâtes papetières.

Le procédé au sulfate de sodium ou procédé Kraft est basé sur l'utilisation de soude et de sulfate de sodium. Le traitement chimique des copeaux de bois se fait à 150-175°C pendant une durée de 1 à 7 heures en fonction du substrat utilisé. Ces pâtes papetières kraft sont produites à partir des biomasses les plus variées, mais plus particulièrement depuis les espèces arborescentes résineuses (softwood tels que les épicéas ou les pins) ou feuillues (hardwood tels que les eucalyptus) ou encore les déchets lignocellulosiques agricoles (paille de blé, riz, etc.). Elles sont partiellement délignifiées au moyen de cuissons à haute température et en présence de soude. Cette délignification est contrôlée par les paramètres opératoires des réacteurs. La cuisson est réalisée dans un réacteur vertical, où les copeaux descendent par gravité et rencontrent les diverses liqueurs de cuisson. Le sulfure de sodium est préparé directement à partir de sulfate de sodium par combustion. Lors de la cuisson, le sulfure de sodium est hydrolysé en soude, en NaHS et en H₂S. Les différents composés soufrés présents réagissent avec la lignine pour donner des thiolignines plus facilement solubles. La liqueur appliquée aux copeaux est appelée liqueur blanche. La liqueur extraite du réacteur ou lessiveur contenant les composés éliminés de la paroi est appelée liqueur noire.

A l'issue de ce prétraitement alcalin, on aboutit à la production d'un substrat prétraité, enrichi en cellulose puisqu'il contient entre 60 et 90% de cellulose et entre 5 et 20% d'hémicellulose. Ce substrat est ensuite partiellement lavé.

D'autres prétraitements alcalins sont étudiés à l'échelle du laboratoire, dans le but de réduire les coûts liés à cette étape dans l'optique d'une production de carburant.

Le prétraitement chimique alcalin réalisé à l'étape A peut également être un prétraitement par explosion des fibres à l'ammoniac, encore appelé prétraitement de AFEX (Ammonia Fiber Explosion) ou un prétraitement par percolation utilisant de l'ammoniaque avec recycle, encore appelé prétraitement ARP (Ammonia Recycle Percolation).

Le procédé ARP (Ammonia Recycle Percolation) est un procédé de prétraitement utilisant de l'ammoniaque en mode recycle. Ce type de procédé est notamment décrit par Kim et al., 2003, Biores. Technol. 90 (2003) p 39-47. La température élevée de la percolation conduit à une solubilisation partielle à la fois de la lignine et des hémicelluloses, cette solution est ensuite chauffée pour recycler l'ammoniaque et récupérer d'une part la lignine extraite, par exemple pour une valorisation énergétique, et d'autre part les sucres solubles issus des hémicelluloses.

Le procédé AFEX (Ammonia Fiber Explosion) consiste à introduire le substrat lignocellulosique dans un cuiseur à haute pression en présence d'ammoniac, puis de provoquer une détente explosive en sortie du réacteur et de recycler l'ammoniac alors sous forme gazeuse. Ce type de procédé est notamment décrit par Teymouri et al., 2005, Biores. Technol. 96 (2005) p.2014-2018. Ce procédé conduit principalement à une déstructuration de la matrice de la biomasse mais il n'y a pas de séparation phasique des composés lignine, hémicellulose et cellulose en sortie de traitement.

D'autres traitements alcalins sont aussi à l'étude, notamment à base de soude ou de chaux, une revue non exhaustive est donnée par Ogier et al., 1999, Oil & Gas Science and Technology - Revue de l'IFP, Vol. 54 (1999), No. 1, pp. 67-94.

Ces prétraitements alcalins peuvent être combinés à une action mécanique, créée par exemple dans une extrudeuse de type bi-vis ou une défribreuse.

L'étape A2, optionnelle,e st une étape consistant à laver le substrat prétraité par introduction d'un ou plusieurs liquides de lavage. Cette étape de lavage peut également se limiter à une étape de dilution.

Le substrat prétraité obtenu, éventuellement lavé, comprend des matières solides insolubles et éventuellement une fraction liquide.

Une mise au pH du substrat issu du prétraitement est nécessaire pour réaliser l'étape d'hydrolyse enzymatique. En effet, l'hydrolyse enzymatique s'effectue dans des conditions douces, à une température de l'ordre de 45-50°C et pH compris entre 4,5 et 5,5. Très préférentiellement, le pH est compris entre 4,8-5,2. Cette mise au pH peut être réalisée séparément ou à l'entrée du réacteur d'hydrolyse enzymatique. La mise au pH est avantageusement réalisée en mélangeant le produit obtenu après l'étape de conversion acide avec le flux comprenant le substrat prétraité, éventuellement complété si nécessaire par l'ajout d'une solution.acide.

Grâce au prétraitement alcalin, l'accessibilité de la cellulose et des hémicelluloses est améliorée et leur susceptibilité à l'hydrolyse enzymatique est excellente. Lors de cette étape de saccharification, les polymères de celluloses et d'hémicelluloses sont convertis en sucres dits "très fermentescibles " (glucose, mannose), "médiocrement fermentescibles" (galactose) et "difficilement fermentescibles" (xylose et arabinose).

L'hydrolyse enzymatique (étape B1) est réalisée au moyen d'enzymes produites par un microorganisme. Des microorganismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases et les hémicellulases, adaptées à l'hydrolyse poussée de la cellulose et des hémicelluloses.

L'étape B2 est l'étape de fermentation alcoolique : les sucres fermentescibles sont ainsi transformés en alcools par des levures. La fermentation est réalisée le plus souvent à une température comprise entre 30 et 35°C. A l'issue de cette étape, on obtient un moût de fermentation comprenant des matières en suspension et une phase liquide dans laquelle se trouve l'alcool produit.

Lorsque les étapes B1 et B2 sont réalisées simultanément dans un même réacteur, on parle de procédé SSF. La température est alors située autour de 35°C.

Lorsqu'on réalise simultanément l'hydrolyse enzymatique des polymères de sucres et la fermentation du glucose et xylose, on parle de procédé SSCF (Simultaneous Saccharification and Co Fermentation). La température est alors située autour de 35°C.

L'étape C est l'étape de séparation/purification du produit qui permet d'obtenir un alcool purifié prêt à être vendu. Cette étape comprend avantageusement trois sous étapes: une étape de distillation permettant d'obtenir un alcool à l'azéotrope, une étape de déshydratation donnant l'alcool déshydraté prêt pour un emploi carburant et une étape de clarification des vinasses permettant de séparer les résidus insolubles des eaux usées.

Selon la quantité de résidus solides, l'étape de séparation est réalisée avant ou après l'étape de distillation.

L'étape D correspond à l'étape de conversion acide et donc de fabrication proprement dite de l'agent anti-bactérien.

La conversion acide (étape D) est effectuée à une température comprise entre 120 et 200°C, pendant une durée de 0,5 à 3 heures, en présence d'un acide fort, de préférence choisi parmi l'acide sulfurique ou phosphorique.

De façon préférée, la conversion acide est effectuée à une température comprise entre 130 et 190°C, pendant une durée d'au plus 1,5 heures, en présence d'une solution de 1 à 8% d'acide sulfurique.

Selon un mode de réalisation du procédé selon l'invention, le liquide contenant les pentoses solubilisés et utilisé lors de l'étape de conversion acide est une fraction liquide du flux de vinasses clarifiées, obtenues à l'issue de l'étape de séparation/purification (étape C).
La fraction liquide mise en jeu dans l'étape de conversion acide représente entre 5 et 75% poids, de préférence entre 10 et 60% poids, du flux des vinasses clarifiées. Selon un autre mode de réalisation du procédé selon l'invention, le liquide contenant les pentoses solubilisés et utilisé lors de l'étape de conversion acide est une fraction du flux contenant au moins le réactif chimique et le substrat prétraité, obtenu après l'étape de prétraitement.

Cette fraction liquide représente entre 10 et 100% poids dudit flux, de préférence entre 20 et 100% poids.

Selon un autre mode de réalisation du procédé selon l'invention, le liquide contenant les pentoses solubilisés et utilisé dans l'étape de conversion acide est une fraction du flux contenant les eaux de lavage usées, obtenu après l'étape de prétraitement. Cette fraction liquide représente entre 10 et 100% poids, de préférence entre 20 et 100% poids dudit flux.

La totalité du produit résultant de l'attaque acide réalisée à l'étape D est mélangée au flux de substrat prétraité, éventuellement lavé, lors de la mise au pH nécessaire pour l'étape d'hydrolyse enzymatique.

La présence de furfural à des concentrations contrôlées, par exemple comprises entre 1 et 5g/L permet de réduire considérablement le développement des contaminants bactériens et en particulier des bactéries lactiques. À ces mêmes concentrations en furfural, la levure alcoolique n'est que faiblement affectée. Les concentrations en furfural sont données par rapport à la fraction de liquide entrant dans les réacteurs d'hydrolyse enzymatique et/ou de fermentation.

La concentration acceptable en furfural, pour laquelle la fonction d'agent antibactérien du furfural est remplie, sans devenir un produit toxique pour la levure est dépendante des conditions de mise en oeuvre du procédé et de la levure utilisée.

Dans le procédé selon l'invention, l'étape d'attaque acide des pentoses produit essentiellement du furfural. Ce dernier constitue donc le composé inhibiteur majoritairement présent, associé éventuellement à des traces (moins de 0,1 g/L) d'acide acétique et formique. À titre de comparaison, il est utile de rappeler que dans les procédés de prétraitement conduits en conditions acides (tels que l'explosion à la vapeur ou la cuisson acide), le furfural n'est que l'un des composés inhibiteurs parmi une cinquantaine de produits obtenus identifiables. Dans ces procédés, il devient donc très difficile de gérer correctement l'action antimicrobienne qui est la résultante des effets isolés ou synergiques des composés issus de la dégradation des sucres ou de la lignine.

Une concentration en furfural obtenu dans les conditions du procédé de la présente invention permet donc de contrôler les populations bactériennes.

Dans un premier temps, la concentration de 1 g/L de furfural est suffisante car les bactéries ne sont pas adaptées à ce produit. Mais rapidement, il est nécessaire augmenter la concentration en furfural bien au-delà de 1 g/L pour réduire l'infection bactérienne et lactique plus particulièrement. Il n'est pas recommandé de dépasser la concentration de 5 g/L qui commence à pénaliser les performances fermentaires des levures du genre saccharomyces" (diminution de la productivité). Cet ajustement en concentration est notamment possible en contrôlant et ajustant les flux qui sont envoyés vers l'étape de conversion acide.

D'autre part, la présence de furfural en quantité contrôlée dans le procédé selon l'invention permet de diminuer la production de glycérol, principal co-produit de la fermentation alcoolique et responsable de la diminution du rendement de conversion des sucres en C6 (glucose et hexoses) en alcool. L'addition de furfural dans le milieu oblige la levure utilisée lors de l'étape de fermentation alcoolique à le réduire en acide furfurylique au moyen de co-facteurs (NADH). Cette réduction est prioritaire par rapport à la formation du glycérol qui classiquement peut être responsable d'une fuite de 5 à 8 % de carbone. En effet, dans un milieu ne contenant pas de furfural, la régénération indispensable du co-facteur est réalisée par la synthèse de glycérol à partir de glucose, affectant par conséquence le rendement de production d'alcool.

Le furfural constitue une protection contre les contaminations bactériennes et notamment lactiques, à des températures variant entre 30 et 70°C, ce qui correspond aux températures auxquelles sont réalisées l'hydrolyse enzymatique (40-50°C) et la fermentation alcoolique (30-35°C).

Le procédé de production d'alcool à partir de biomasse lignocellulosique va être détaillé ci-dessous en faisant référence aux figures.
La Figure 1 représente un schéma du procédé de l'invention selon le premier mode de réalisation, dans lequel la fraction liquide comprenant les pentoses qui subit la conversion acide provient des vinasses clarifiées.
La Figure 2 représente un schéma du procédé de l'invention selon le deuxième mode de réalisation dans lequel la fraction liquide comprenant les pentoses qui subit la conversion acide est issue de la solution alcaline usée obtenue en sortie du prétraitement après séparation.

Les postes A à D représentés sur les figures correspondent respectivement aux différentes étapes du procédé de la présente invention, désignées par les lettres identiques

Le fonctionnement du poste A est notamment dépendant du prétraitement considéré.

Dans le cas d'un prétraitement dit Kraft, la biomasse est introduite par une conduite 1 dans le réacteur de cuisson ou lessiveur (A1). Une solution alcaline, encore appelée liqueur blanche, y est également introduite via la conduite 2. La biomasse est partiellement délignifiée au moyen de cuissons à haute température et en présence de soude. La lignine solubilisée est retirée avec la solution alcaline et est évacuée par la conduite 3 avec la solution alcaline usée, aussi appelée liqueur noire.

Cette étape de délignification peut avoir lieu dans plusieurs lessiveurs successifs non représentés sur les figures et est contrôlée par les paramètres opératoires fixés dans ces dispositifs.

La pâte obtenue en sortie des lessiveurs circulant dans la conduite 4 et correspondant au substrat prétraité est enrichie en cellulose : elle contient entre 60 et 90 % en poids de cellulose par rapport à la matière solide totale. Elle est envoyée vers le réacteur A2 pour subir une étape de lavage : un ou plusieurs liquides de lavage peuvent être introduits via une conduite 5. Cette étape de lavage est suivie d'une étape de mise au pH de la pâte, en vue de l'étape d'hydrolyse enzymatique (étape B). Les liquides de lavage usés sont retirés par la conduite 6.

Dans le cas d'un traitement AFEX, la biomasse est introduite par la conduite 1 dans un réacteur de cuisson (A1). Une solution ammoniacale est introduite par la conduite 2 sous pression, de 15 à 30 bars, à température modérée (70°C à 110°C). Le mélange est maintenu dans ces conditions durant un temps déterminé en fonction du substrat, puis détendu en sortie du cuiseur. La solution ammoniacale est récupérée via la conduite 3 sous forme gazeuse pour être recyclée. La substrat prétraité extrait par la conduite 4 du cuiseur a essentiellement la même composition que le substrat en entrée. L'étape de lavage A2 après ce prétraitement peut se limiter à une étape de dilution avec un liquide de dilution introduit par la conduite 5, auquel cas le flux de liquide de lavage évacué par la conduite 6 est nul. L'étape de mise au pH est ensuite réalisée, en vue de l'étape d'hydrolyse enzymatique.

Dans le cas d'un prétraitement ARP, la biomasse est introduite par la conduite 1 dans un réacteur de cuisson (A1). Une solution ammoniacale est percolée sur la biomasse sous pression (15 à 30 bars) et à température élevée, 130°C à 190°C. La biomasse est partiellement délignifiée, une partie des hémicelluloses est aussi solubilisée. Les sucres et la lignine solubilisés sont retirés avec la solution alcaline usée et sont évacués par la conduite 3. Le substrat prétraité issu de la cuisson et extrait par la conduite 4 est lavé dans un réacteur de lavage (A2): un ou plusieurs liquides de lavage peuvent être introduits par la conduite 5. Cette étape de lavage est suivie d'une étape de mise au pH du substrat en vue de l'hydrolyse enzymatique. Les liquides de lavage usés sont retirés via la conduite 6.

A l'issue du prétraitement, le substrat circulant dans la conduite 7 contient entre 1 et 55 % poids et préférentiellement entre 1 et 45 % poids de matière solide insoluble et est envoyé vers les étapes de conversion en alcool, comprenant au moins une étape d'hydrolyse enzymatique (étape B1) et au moins une étape de fermentation alcoolique (étape B2).

Dans l'étape d'hydrolyse enzymatique B1, une solution enzymatique contenant des enzymes cellulolytiques et/ou hémicellulolytiques est introduite par la conduite 8 et mélangée avec le substrat prétraité, éventuellement lavé circulant dans la conduite 7.

L'hydrolysat obtenu à l'issue de l'étape B1 évacué par la conduite 9 est constitué de pentoses (xylose et arabinose principalement), de disaccharides (cellobiose) et d'hexoses (galactose, mannose et surtout glucose). Il est ensuite envoyé vers le poste B2, où a lieu la fermentation alcoolique, en présence de levures ou autres microorganismes introduits par la conduite 10.

La fraction obtenue après fermentation est envoyée par la conduite 11 vers le poste C où a lieu l'étape de séparation/purification. On sépare les résidus solides, l'alcool produit et les vinasses clarifiées.

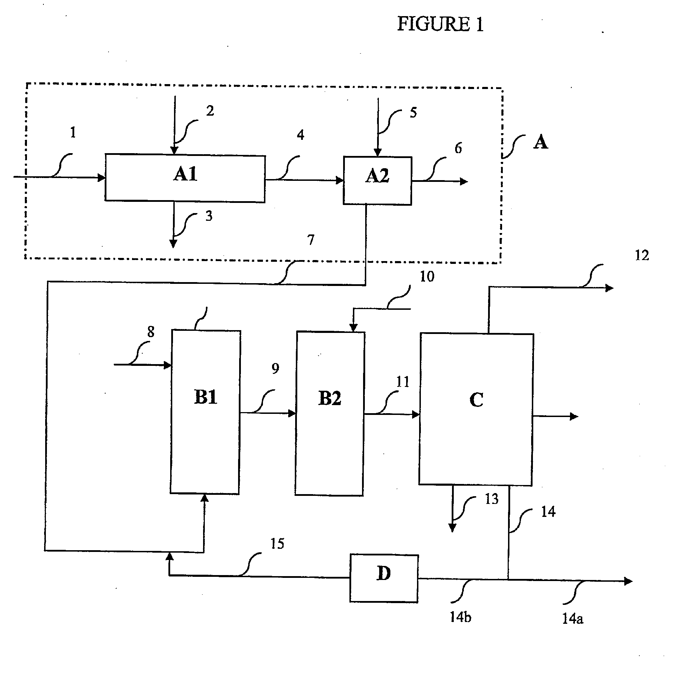

Selon le mode de réalisation représenté sur la Figure 1, à l'issue de l'étape C de séparation/purification, tous les hexoses (glucose et mannose) qui étaient présents dans le flux sortant de l'hydrolyse enzymatique ont été convertis en alcool et sont extraits par la conduite 12. Les pentoses ne sont pas utilisés puisqu'ils font partie des sucres "difficilement fermentescibles" et se retrouvent dans les vinasses clarifiées extraites par la conduite 14 par exemple en pied de colonne de distillation. Le principal pentose présent dans les vinasses est le xylose. Par exemple, pour un moût de 100 g/L de glucose, et en fonction de la source de végétal, la concentration en xylose peut être comprise entre 5 et 55 g/L.

Le flux de vinasses clarifiées est séparé alors en deux flux circulant respectivement dans les conduites 14a et 14b. Une partie du flux représentant entre 5 et 75% poids du flux total de vinasses est dirigée dans la conduite 14b vers un réacteur de conversion acide (poste D). Par chauffage à chaud, une fraction des pentoses contenus dans ce flux et qui dépend des conditions opératoires choisies est ainsi convertie spécifiquement en furfural. Le flux de produit obtenu après l'attaque acide est évacué via la conduite 15 .

Par exemple, si la cuisson acide est relativement modérée, (120°C, 1,5 heure, 4% d'acide sulfurique), près de 20% des pentoses peuvent être convertis en furfural sans que le furfural ne soit lui même dégradé en sous produits. Tout au plus, 2 à 3 % du furfural peut être converti en acide formique et/ou polymérisation du furfural.

Le produit issu de l'attaque acide et circulant dans la conduite 15 obtenu et comprenant le furfural est ainsi avantageusement mélangé au flux circulant dans la conduite 7 constitué du végétal prétraité avant d'être envoyé vers l'étape d'hydrolyse enzymatique.

L'autre partie du flux de vinasses est extraite par la conduite 14a pour être traitée en vue de la valorisation des pentoses.

L'alcool produit est extrait par la conduite 12 et les résidus solides obtenus après l'étape de séparation/purification (étape C) sont évacués par la conduite 13.

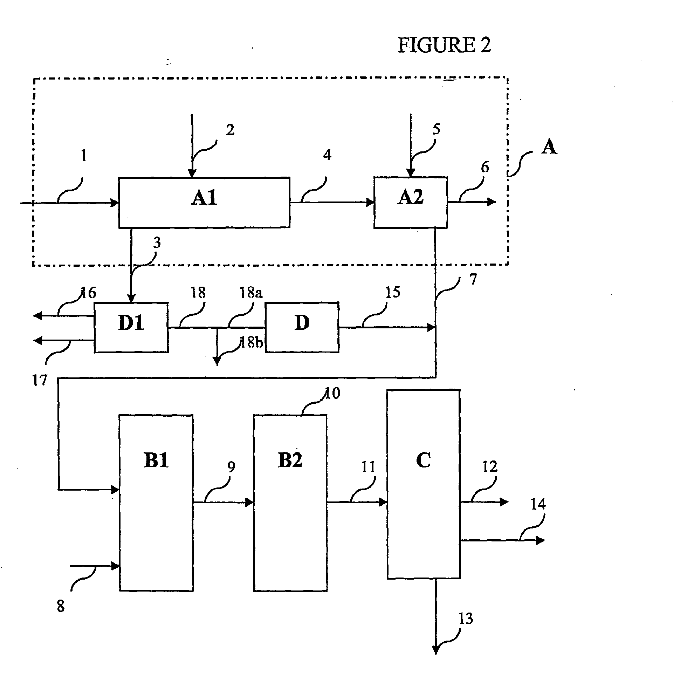

Le mode de réalisation représenté sur la Figure 2, correspond plus particulièrement à une mise en oeuvre du procédé selon la présente invention dans lequel le prétraitement réalisé est un prétraitement de type ARP auquel on associe une étape de régénération de la solution ammoniacale.

La biomasse est introduite par la conduite 1 dans un réacteur de cuisson (A1). Une solution ammoniacale est percolée sur la biomasse sous pression (15 à 30 bars) et à température élevée, 130°C à 190°C. La biomasse est partiellement délignifiée, une partie des hémicelluloses est aussi solubilisée. Les sucres et la lignine solubilisés sont retirés avec la solution alcaline usée et sont évacués par la conduite 3. Une étape (D1) de régénération de la solution ammoniacale peut être ajoutée, qui permet de générer au moins deux flux et préférentiellement au moins trois flux : un premier flux circulant dans la conduite 18 constitué de tout ou partie des sucres solubilisés en solution, un second flux circulant dans la conduite 16 contenant la solution alcaline. La lignine peut être contenue dans le deuxième flux circulant dans la conduite 16 ou préférentiellement dans un flux séparé évacué par une conduite 17. Dans le cas d'un prétraitement de type ARP, cette régénération est une évaporation de la solution ammoniacale.

Les sucres issus des hémicelluloses sont récupérés dans un flux circulant dans la conduite 18. Ce flux contient des matières solubles qui sont principalement les pentoses et les hexoses issus des hémicelluloses, et en plus faible quantité les acides organiques issus de la dégradation partielle de la lignine.

Dans certains cas, en fonction de la composition des hémicelluloses du substrat initial, la proportion d'hexoses dans le flux de la conduite 18 peut représenter jusqu'à 70% des sucres solubilisés. Toutefois, la mise en oeuvre selon la figure 2 est préférentiellement utilisée pour des substrats où la proportion d'hexoses dans les hémicelluloses est inférieure à 20% et plus préférentiellement inférieure à 10% des hémicelluloses.

Dans une variante de la mise en oeuvre correspondant à la Figure 2, non représentée, le poste de séparation D1 des sucres de la solution alcaline peut être placé sur le flux des liquides de lavage du substrat prétraité circulant dans la conduite 6.

Une partie représentant 5 à 100% poids du flux circulant dans la conduite 18 (flux 18a) est dirigée vers un réacteur de conversion acide. Par chauffage à chaud, une fraction des pentoses est ainsi convertie spécifiquement en furfural. Le flux de produit issu de l'attaque acide est évacué par la conduite 15. Dans les conditions de chauffage acides précédemment décrites, le produit issu de la réaction est essentiellement du furfural. Il n'est toutefois pas exclu d'obtenir de faibles quantités de 5-HMF et d'acide lévulinique et formique issus de la dégradation des traces d'hexoses.

La fraction 18b qui n'est pas envoyée vers le réacteur de conversion acide est traitée en vue de la valorisation de ces sucres.

Le flux sortant du réacteur de conversion acide par la conduite 15 peut alors être mélangé au flux 7 sortant de l'étape de prétraitement et destiné à être envoyé dans le réacteur d'hydrolyse enzymatique (étape B1).

Ainsi le flux de solution acide comprenant l'agent antimicrobien contribue également à la mise au pH du flux issu du prétraitement chimique alcalin, préalablement à l'étape d'hydrolyse enzymatique.

Les exemples ci-après illustrent l'invention, sans en limiter la portée.

### Exemple 1

On considère un procédé de production d'éthanol à partir de pâte papetière de type "Kraft". La pâte est obtenue à partir de bois "softwood" prétraité dans un procédé papetier type Kraft, qui est un procédé alcalin en présence de soude. La pâte pauvre en lignine issue du Kraft est ensuite lavée et neutralisée, puis introduite dans le procédé de conversion de substrat cellulosique en éthanol par hydrolyse enzymatique et fermentation du glucose et du mannose (sucres à 6 atomes de carbones)

Le procédé traite 40 tonnes / heure de pâte (base matière sèche). La composition de la matière sèche est la suivante:

| | |
|---|---|
| Cellulose (%) | 77,75% |
| Xylanes (%) | 8,96% |
| Mannanes (%) | 5,26% |
| Autres (%) | 7,68% |

Le procédé de conversion en éthianol contient les étapes suivantes: hydrolyse enzymatique, fermentation alcoolique, distillation et déshydratation de l'éthanol, séparation des résidus solides des vinasses.

L'hydrolyse enzymatique s'opère à pH 5, avec un flux d'entrée contenant 10% de matière sèche. Dans les conditions d'hydrolyse choisies, 90% des polymères de sucres sont solubilisés en monomères.

Le jus sucré est ensuite envoyé en fermentation où 90% des sucres glucose et mannose sont convertis en éthanol.

Le vin est ensuite envoyé en distillation, qui contient une étape de stripping (distillation en présence de solides) suivie d'une étape de rectification pour donner un mélange éthanol-eau à l'azéotrope. Le mélange obtenu eau-éthanol est ensuite déshydraté sur tamis moléculaire pour obtenir l'éthanol recherché.

Les vinasses sont envoyées vers une centrifugeuse pour séparer les phases solide et liquide.
45% du flux de phase liquide obtenu après séparation liquide est envoyé dans le réacteur de cuisson acide. Le temps de cuisson est de 60 minutes à 180°C en présence de 2% poids de H₂SO₄ en solution, ce qui représente un débit 3200 kg / h.

Dans ces conditions, 60% des xyloses présents sont convertis en furfural.

Cette solution est ensuite refroidie et mélangée avec la pâte papetière avant l'hydrolyse enzymatique. Ceci conduit à la présence de 2 g / litre de furfural dans le milieu, ce qui offre une protection antibactérienne lors de l'hydrolyse et la fermentation.

### Exemple 2

On considère un procédé de production d'éthanol à partir de pâte papetière de type "Kraft". La pâte est obtenue à partir de bois "hardwood" prétraité dans un procédé papetier type Kraft, qui est un procédé alcalin en présence de soude. La pâte pauvre en lignine issue du Kraft est ensuite lavée et neutralisée, puis introduite dans le procédé de conversion de substrat cellulosique en éthanol par hydrolyse enzymatique et fermentation du glucose.

Le procédé traite 18 tonnes / heure de pâte (base matière sèche). La composition de la matière sèche est comme suit:

| | |
|---|---|
| Cellulose (%) | 72% |
| Xylanes (%) | 15,9% |
| Mannanes(%) | 0% |
| Autres (%) | 12,1% |

Le procédé de conversion en éthanol contient les étapes suivantes : hydrolyse enzymatique, fermentation alcoolique, distillation et déshydratation de l'éthanol, séparation des résidus solides des vinasses.

L'hydrolyse enzymatique s'opère à pH 5, avec un flux d'entrée contenant 20% de matière sèche. Dans les conditions d'hydrolyse choisies, 90% des polymères de sucres sont solubilisés en monomères.

Le jus sucré est ensuite envoyé en fermentation où 90% du glucose est converti en éthanol. Le vin est ensuite envoyé en distillation, qui contient une étape de stripping (distillation en présence de solides) suivie d'une étape de rectification pour donner un mélange éthanol-eau à l'azéotrope. Ce mélange est ensuite déshydraté sur tamis moléculaire et on obtient l'éthanol recherché.

Les vinasses sont envoyées vers une centrifugeuse pour séparer les phases solide et liquide. 15% du flux de phase liquide obtenue après séparation est envoyé dans le réacteur de cuisson acide. Le temps de cuisson est de 60 minutes à 180°C en présence de 2%poids de H₂SO₄ en solution, ce qui représente un débit 205 kg / h. Dans ces conditions, 60% des xyloses présents sont convertis en furfural. Cette solution est ensuite refroidie et mélangée avec la pâte papetière avant l'hydrolyse enzymatique.

Ceci conduit à la présence de 2 g / litre de furfural dans le milieu, ce qui offre une protection antibactérienne lors de l'hydrolyse et la fermentation.

### Exemple 3

On considère un procédé de production d'éthanol à partir de paille de blé. La paille est prétraitée par un procédé type AFEX, qui est un procédé alcalin en-présence d'ammoniac. Le substrat prétraité est converti en éthanol par hydrolyse enzymatique et fermentation du glucose.

Le procédé traite 120 tonnes / heure de paille (base matière sèche). La composition de la matière sèche est comme suit:

| | |
|---|---|
| Cellulose (%) | 43% |
| Xylanes (%) | 26% |
| Mannanes (%) | 0% |
| Autres (%) | 31% |

Le traitement AFEX ne solubilise aucune fraction du substrat, et l'essentiel du catalyseur chimique est recyclé en phase gazeuse, le substrat prétraité a donc une composition très similaire à la paille entrante.

Le procédé de conversion en éthanol contient les étapes suivantes: hydrolyse enzymatique, fermentation alcoolique, distillation et déshydratation de l'éthanol, séparation des résidus solides des vinasses.

L'hydrolyse enzymatique s'opère à pH 5, avec un flux d'entrée contenant 20% de matière sèche. Dans les conditions d'hydrolyse choisies, 80% des polymères de sucres sont solubilisés en monomères.

Le jus sucré est ensuite envoyé en fermentation où 90% du glucose est converti en éthanol.

Le vin est ensuite envoyé en distillation, qui contient une étape de stripping (distillation en présence de solides) suivie d'une étape de rectification pour donner un mélange éthanol-eau à l'azéotrope. Ce mélange est ensuite déshydraté sur tamis moléculaire et on obtient l'éthanol recherché.

Les vinasses sont envoyées vers une centrifugeuse pour séparer les phases solide et liquide. 10% du flux de phase liquide obtenue après séparation est envoyé dans le réacteur de cuisson acide. Le temps de cuisson est de 60 minutes à 180°C en présence de 2%poids de H₂SO₄ en solution, ce qui représente un débit 906 kg / h.

Dans ces conditions, 60% des xyloses présents sont convertis en furfural. Cette solution est ensuite refroidie et mélangée avec la paille prétraitée avant l'hydrolyse enzymatique.

Ceci conduit à la présence de 2 g / litre de furfural dans le milieu, ce qui offre une protection antibactérienne lors de l'hydrolyse et la fermentation.

### Exemple 4:

On considère un procédé de production d'éthanol à partir de paille de blé. La paille est prétraitée par un procédé type ARP, qui est un procédé alcalin de percolation d'une solution ammoniacale. Le substrat prétraité est converti en éthanol par SSCF *(Simultaneous Saccharification and Co Fermentation).*

La SSCF est une étape dans laquelle on réalise simultanément une hydrolyse enzymatique des polymères de sucres et la fermentation du glucose et du xylose (par une levure *S. cerevisae* génétiquement modifiée pour utiliser les sucres en C5).

Le procédé traite 70 tonnes par heure de paille (base matière sèche). La composition de la matière sèche est la suivante:

| | |
|---|---|
| Cellulose (%) | 43% |
| Xylanes (%) | 26% |
| Mannanes (%) | 0% |
| Autres (%) | 31% |

Le traitement ARP consiste à percoler une solution ammoniacale sur le substrat, ce qui conduit à une déligninification partielle du substrat ainsi qu'à une solubilisation d'une partie des hémicelluloses. La cellulose n'est pas ou très peu solubilisée (moins de 1%). Dans les conditions de température et ratio liquide choisi, 80% de la lignine est retirée et 51% des hémicelluloses sont solubilisées.

55% de la matière solubilisée est retirée avec la solution ammoniacale usée. Les 45% restant sont retirés lors des étapes successives de lavage du substrat prétraité.

La solution ammoniacale usée est envoyée dans un évaporateur afin de récupérer le réactif en phase gazeuse, puis dans un réacteur où la lignine est recondensée. Une séparation solide - liquide en sortie de ce réacteur permet de récupérer 75% des sucres envoyés. 76% de ce flux est envoyé dans le réacteur de cuisson acide. Le temps de cuisson est de 60 minutes à 180°C en présence de 2%poids de H₂SO₄ en solution, ce qui représente un débit 874 kg / h.

Dans ces conditions, 60% des xyloses présents sont convertis en furfural. Cette solution est ensuite refroidie et mélangée avec la paille prétraitée avant l'hydrolyse enzymatique.

Le procédé de conversion en éthanol contient les étapes suivantes: SSCF, distillation et déhydratation de l'éthanol, séparation des résidus solides des vinasses.

La SSCF s'opère à pH 4.5, avec un flux d'entrée contenant 8% de matière solide. Dans les conditions d'hydrolyse et de fermentation choisies, 85% des polymères de sucres sont solubilisés en monomères, puis directement fermentés en éthanol. 90% des sucres en C6 sont convertis, et 70% des sucres en C5.

Le vin est ensuite envoyé en distillation, qui contient une étape de stripping (distillation en présence de solides) suivie d'une étape de rectification pour donner un mélange éthanol-eau à l'azéotrope. Ce mélange est ensuite déshydraté sur tamis moléculaire et on obtient l'éthanol recherché.

Les vinasses sont envoyées vers une centrifugeuse pour séparer les phase solide et liquide.

Le mélange du liquide issu de la cuisson acide avec la paille prétraitée avant l'étape de SSCF conduit à la présence de 2 g / litre de furfural dans le milieu, ce qui offre une protection antibactérienne lors de l'étape combinée d'hydrolyse et de fermentation.

## Revendications

1. Procédé de production d'alcool à partir d'un substrat cellulosique ou lignocellulosique dans lequel on produit du furfural et qui comprend au moins les étapes suivantes :
- une première étape de prétraitement chimique alcalin (étape A) dudit substrat, comprenant une étape de chauffage A1 en présence d'un réactif chimique alcalin, à l'issue de laquelle on obtient au moins un flux comprenant au moins ledit réactif chimique et le substrat prétraité et éventuellement une étape A2 de lavage dudit substrat prétraité, à l'issue de laquelle on obtient le substrat prétraité lavé et au moins un flux contenant les eaux de lavage usées, puis
- une étape de mise au pH entre 4,5 et 5,5 du substrat prétraité éventuellement lavé couplée à une étape d'hydrolyse enzymatique (étape B1) dudit substrat prétraité mettant en jeu des enzymes cellulolytiques et/ou hémicellulolytiques,
- une étape de fermentation alcoolique (étape B2) de l'hydrolysat obtenu par un microorganisme alcooligène, à l'issue de laquelle on obtient un moût de fermentation comprenant des matières en suspension et une phase liquide comprenant un alcool et des vinasses, puis
- une étape de séparation/purification (étape C) à l'issue de laquelle on obtient l'alcool purifié, les vinasses clarifiées, et un résidu insoluble, et
- une étape de conversion acide (étape D) d'au moins un des liquides obtenus lors d'une des étapes précédentes et contenant des pentoses solubilisés, une partie desdits pentoses étant convertis en furfural, et
- une étape de mélange du furfural obtenu lors de l'étape de conversion acide avec le substrat prétraité éventuellement lavé lors de l'étape de mise au pH, la quantité de furfural mélangée étant comprise entre 1 et 5g/l par rapport à la quantité totale de liquide mise en jeu dans l'étape d'hydrolyse enzymatique et/ou de fermentation alcoolique.

2. Procédé selon la revendication 1 dans lequel le prétraitement chimique alcalin est un prétraitement au sulfate de sodium, encore appelé procédé Kraft à l'issue duquel on obtient des pâtes papetières..

3. Procédé selon la revendication 1 dans lequel le prétraitement chimique alcalin est un prétraitement par explosion des fibres à l'ammoniac, encore appelé prétraitement AFEX ou un prétraitement par percolation utilisant de l'ammoniaque avec recycle, encore appelé prétraitement ARP.

4. Procédé selon l'une des revendications précédentes dans lequel le liquide contenant les pentoses solubilisés et utilisé lors de l'étape de conversion acide est une fraction du flux de vinasses clarifiées obtenues à l'issue de l'étape de séparation/purification.

5. Procédé selon la revendication 4 dans lequel ladite fraction représente entre 5 et 75% poids du flux de vinasses clarifiées.

6. Procédé selon l'une des revendications 1 à 3 dans lequel dans lequel le liquide contenant les pentoses solubilisés et utilisé lors de l'étape de conversion acide est une fraction du flux contenant au moins le réactif chimique et le substrat prétraité, obtenu après l'étape de prétraitement.

7. Procédé selon la revendication 6 dans lequel ladite fraction représente entre 10 et 100% poids dudit flux.

8. Procédé selon l'une des revendications 1 à 3 dans lequel dans lequel le liquide contenant les pentoses solubilisés et utilisé lors de l'étape de conversion acide est une fraction du flux contenant les eaux de lavage usées, obtenu après l'étape de prétraitement.

9. Procédé selon la revendication 8 dans lequel ladite fraction représente entre 10 et 100% poids dudit flux.

10. Procédé selon l'une des revendications précédentes dans lequel l'étape de conversion acide est effectuée à une température comprise entre 120 et 200°C, pendant une durée de 0,5 à 3 heures, en présence d'un acide fort de préférence choisi parmi l'acide sulfurique ou phosphorique.

11. Procédé selon la revendication 10 dans lequel l'étape de conversion acide est effectuée à une température comprise entre 130 et 190°C, pendant une durée d'au plus 1,5 heures, en présence d'une solution de 1 à 8% d'acide sulfurique.

## Patentansprüche

1. Verfahren zur Alkoholerzeugung ausgehend von einem cellulosehaltigen oder lignocellulosehaltigen Substrat, in welchem Furfural erzeugt wird und welches mindestens die folgenden Schritte umfasst:
- einen ersten Schritt der alkalischen chemischen Vorbehandlung (Schritt A) des Substrats, welcher einen Schritt des Erwärmens A1 in Gegenwart eines alkalischen chemischen Reagenzes umfasst, nach Abschluss dessen mindestens ein Stoffstrom erhalten wird, der mindestens das chemische Reagenz und das vorbehandelte Substrat umfasst, sowie möglicherweise einen Schritt A2 des Waschens des vorbehandelten Substrats, nach Abschluss dessen ein gewaschenes vorbehandeltes Substrat und mindestens ein Stoffstrom, der das verbrauchte Waschwasser enthält, erhalten werden, und danach
- einen Schritt, in welchem der pH-Wert des vorbehandelten und möglicherweise gewaschenen Substrats auf einen Wert zwischen 4,5 und 5,5 eingestellt wird und welcher mit einem Schritt der enzymatischen Hydrolyse (Schritt B1) des vorbehandelten Substrats gekoppelt ist, wobei cellulosespaltende und/oder hemicellulosespaltende Enzyme zum Einsatz kommen,
- einen Schritt der alkoholischen Vergärung (Schritt B2) des erhaltenen Hydrolysats mittels eines alkoholerzeugenden Mikroorganismus, wobei nach dessen Abschluss eine Gärungsmaische erhalten wird, die Schwebstoffe und eine Flüssigphase umfasst, welche einen Alkohol und Vinassen umfasst, und danach
- einen Schritt der Auftrennung/Aufreinigung (Schritt C), nach Abschluss dessen ein aufgereinigter Alkohol, geklärte Vinassen und ein unlöslicher Rückstand erhalten werden, und
- einen Schritt, in welchem mindestens eine der Flüssigkeiten, die bei einem der vorhergehenden Schritte erhalten wurden und in Lösung gebrachte Pentosen enthalten, im sauren Milieu umgewandelt wird (Schritt D), wobei ein Teil dieser Pentosen in Furfural umgewandelt wird, und
- einen Schritt, in welchem das Furfural, das bei dem Schritt der Umwandlung im sauren Milieu erhalten wurde, dem Substrat beigemischt wird, das beim Schritt der pH-Einstellung vorbehandelt und möglicherweise gewaschen wird, wobei die Menge an beigemischtem Furfural im Bereich von 1 bis 5 g/l liegt, bezogen auf die Gesamtmenge an Flüssigkeit, die in dem Schritt der enzymatischen Hydrolyse und/oder alkoholischen Vergärung zum Einsatz gebracht wird.

2. Verfahren nach Anspruch 1, wobei es sich bei der alkalischen chemischen Vorbehandlung um eine Vorbehandlung mit Natriumsulfat handelt, die auch als Kraft-Aufschluss bezeichnet wird, und nach deren Abschluss Zellstoff für die Papierherstellung erhalten wird.

3. Verfahren nach Anspruch 1, wobei es sich bei der alkalischen chemischen Vorbehandlung um eine ammoniakbasierte Vorbehandlung zum Faseraufschluss handelt, welche auch als AFEX-Vorbehandlung bezeichnet wird, oder um eine Perkolationsvorbehandlung unter Verwendung zurückgeführten Ammoniak, welche auch als ARP-Behandlung bezeichnet wird.

4. Verfahren nach einem der vorhergehenden Ansprüche, wobei es sich bei der Flüssigkeit, welche die in Lösung gebrachten Pentosen enthält und beim Schritt der Umwandlung im sauren Milieu verwendet wird, um eine Fraktion des Stoffstroms an geklärten Vinassen handelt, der nach Abschluss des Auftrennungs-/Aufreinigungsschrittes erhalten wird.

5. Verfahren nach Anspruch 4, wobei diese Fraktion zwischen 5 und 75 Gewichts-% des Stoffstroms an geklärten Vinassen ausmacht.

6. Verfahren nach einem der Ansprüche 1 bis 3, wobei es sich bei der Flüssigkeit, welche die in Lösung gebrachten Pentosen enthält und beim Schritt der Umwandlung im sauren Milieu verwendet wird, um eine Fraktion des Stoffstroms handelt, der mindestens ein chemisches Reagenz und das vorbehandelte Substrat enthält, wobei er nach dem Vorbehandlungsschritt erhalten wird.

7. Verfahren nach Anspruch 6, wobei diese Fraktion zwischen 10 und 100 Gewichts-% dieses Stoffstroms ausmacht.

8. Verfahren nach einem der Ansprüche 1 bis 3, wobei es sich bei der Flüssigkeit, weiche die in Lösung gebrachten Pentosen enthält und beim Schritt der Umwandlung im sauren Milieu verwendet wird, um eine Fraktion des Stoffstroms handelt, der das verbrauchte Waschwasser enthält, wobei er nach dem Vorbehandlungsschritt erhalten wird.

9. Verfahren nach Anspruch 8, wobei diese Fraktion zwischen 10 und 100 Gewichts-% dieses Stoffstroms ausmacht.

10. Verfahren nach einem der vorhergehenden Ansprüche, wobei der Schritt der Umwandung im sauren Milieu bei einer Temperatur im Bereich von 120 bis 200 °C, während einer Zeitdauer von 0,5 bis 3 Stunden, in Gegenwart einer starken Säure durchgeführt wird, die vorzugsweise aus Schwefel- oder Phosphorsäure ausgewählt ist.

11. Verfahren nach Anspruch 10, wobei der Schritt der Umwandlung im sauren Milieu bei einer Temperatur im Bereich von 130 bis 190 °C, während einer Zeitdauer von mehr als 1,5 Stunden, in Gegenwart einer Lösung von 1 bis 8 % an Schwefelsäure durchgeführt wird.

## Claims

1. A process for the production of alcohol from a cellulosic or lignocellulosic substrate in which furfural is produced and which comprises at least the following steps:
• a first step for alkaline chemical pre-treatment (step A) of said substrate, comprising a step A1 for heating in the presence of an alkaline chemical reagent, at the end of which at least one stream comprising at least said chemical reagent and the pre-treated substrate are obtained, and optionally a step A2 for washing said pre-treated substrate, at the end of which the pre-treated washed substrate and at least one stream containing spent washing water are obtained; then
• a step for bringing the pre-treated and optionally washed substrate to a pH in the range 4.5 to 5.5, coupled with a step for enzymatic hydrolysis (step B1) of said pre-treated substrate using cellulolytic and/or hemicelluloytic enzymes;
• a step (step B2) for alcoholic fermentation of the hydrolysate obtained with an alcohol-producing microorganism, at the end of which a fermentation mash is obtained comprising material in suspension and a liquid phase comprising an alcohol and vinasses; then
• a step (step C) for separation/purification, at the end of which purified alcohol, clarified vinasses and an insoluble residue are obtained; and
• a step (step D) for acid conversion of at least one of the liquids obtained during one of the preceding steps and containing dissolved pentoses, a portion of said pentoses being converted into furfural; and
• a step for mixing furfural obtained during the acid conversion step with the pre-treated substrate optionally washed during the pH adjusting step, the quantity of furfural mixed in being in the range 1 to 5 g/L with respect to the total quantity of liquid employed in the enzymatic hydrolysis step and/or the alcoholic fermentation step.

2. A process according to claim 1, in which the alkaline chemical pre-treatment is a sodium sulphate pre-treatment, also known as the Kraft process, at the end of which paper pulp is obtained.

3. A process according to claim 1, in which the alkaline chemical pre-treatment is an ammonia fiber explosion pre-treatment, also termed AFEX pre-treatment, or an ammonia recycle percolation pre-treatment, also termed ARP pre-treatment.

4. A process according to one of the preceding claims, in which the liquid containing the dissolved pentoses used during the acid conversion step is a fraction of the clarified vinasses stream obtained at the end of the separation/purification step.

5. A process according to claim 4, in which said fraction represents in the range 5% to 75% by weight of the stream of clarified vinasses.

6. A process according to one of claims 1 to 3, in which the liquid containing the dissolved pentoses used during the acid conversion step is a fraction of the stream containing at least the chemical reagent and the pre-treated substrate obtained after the pre-treatment step.

7. A process according to claim 6, in which said fraction represents in the range 10% to 100% by weight of said stream.

8. A process according to one of claims 1 to 3, in which the liquid containing the dissolved pentoses used during the acid conversion step is a fraction of the stream containing the spent washing water, obtained after the pre-treatment step.

9. A process according to claim 8, in which said fraction represents 10% to 100% by weight of said stream.

10. A process according to one of the preceding claims, in which the acid conversion step is carried out at a temperature in the range 120°C to 200°C, for a period of 0.5 to 3 hours, in the presence of a strong acid, preferably selected from sulphuric acid and phosphoric acid.

11. A process according to claim 10, in which the acid conversion step is carried out at a temperature in the range 130°C to 190°C, for a period of at most 1.5 hours, in the presence of a 1% to 8% sulphuric acid solution.
